## Europäisches Patentamt
## European Patent Office
## Office européen des brevets

(11) Publication number : **0 289 198 B1**

# (12) EUROPEAN PATENT SPECIFICATION

(45) Date of publication of patent specification :
**12.02.92 Bulletin 92/07**

(51) Int. Cl.⁵ : **A44B 18/00**

(21) Application number : **88303529.7**

(22) Date of filing : **20.04.88**

(54) **Loop fastening material for fastening device and method of making same.**

(30) Priority : **24.04.87 US 40520**

(43) Date of publication of application :
**02.11.88 Bulletin 88/44**

(45) Publication of the grant of the patent :
**12.02.92 Bulletin 92/07**

(84) Designated Contracting States :
**AT BE CH DE ES FR GB GR IT LI LU NL SE**

(56) References cited :
**EP-A- 0 233 364**
**FR-A- 1 375 254**
**US-A- 3 577 607**
**US-A- 3 715 415**
**US-A- 3 943 981**

(73) Proprietor : **THE PROCTER & GAMBLE
COMPANY
One Procter & Gamble Plaza
Cincinnati Ohio 45202 (US)**

(72) Inventor : **Noel, John Richard
1526 Shenandoah Avenue
Cincinnati Ohio 45237 (US)**
Inventor : **Scripps, Charles Locke
1280 Barrington Woods Drive
Brookfield, WI 53005 (US)**

(74) Representative : **Gibson, Tony Nicholas et al
Procter & Gamble (NTC) Limited Whitley Road
Longbenton Newcastle upon Tyne NE12 9TS
(GB)**

## Description

## FIELD OF THE INVENTION

The present invention relates to a loop fastening material for fastening devices and, more particularly, to a low-cost loop fastening material and a method for producing such a loop fastening material from an orientable material and filaments.

## BACKGROUND OF THE INVENTION

Fastening devices such as hook and loop-type fasteners are known and have gained wide acceptance. Such materials are generally known by the tradename 'Velcro' and are generally described in U.S. Patent 2,717,437; U.S. Patent 3,009,235; U.S. Patent 3,266,113; U.S. Patent 3,550,837; and U.S. Patent 4,169,303. The hook and loop-type fastener comprises two mating fastening materials wherein a hook fastening material engages a loop fastening material. Engagement of the complementary mating hook and loop fastening materials will occur by placing the surface defined by the hook in face to face relationship with the surface defined by the loop. The fastener resists separation by shear stress and certain peel forces applied to the fastener during use but are readily separable by peel forces applied substantially normal to the plane of their engagement.

One such fastening device incorporating mating hooks and loops is disclosed in US-A-3577607 in which a heat-shrinkable woven backing material is interwoven with non-heat shrinkable continuous filaments. The patent describes a method for producing a loop component of a hook and loop fastening device for a disposable garment, said loop component having a multiplicity of fibrous elements projecting from a backing of orientable material capable of exhibiting a dimensionally unstable state and a dimensionally stable state, said backing having a predominant response direction in which the orientable material contracts from its unstable state to its stable state, said method comprising the steps of:

(a) providing a backing of orientable material, in its unstable state;

(b) positioning a plurality of filaments on said backing so as to support and hold said filaments on said backing at spaced support regions disposed along each of said filaments, adjacent support regions of each filament defining therebetween an unsupported portion; and

(c) causing said orientable material to contract in its reponse direction to its stable state, thereby shirring said filaments at said unsupported portions to form fibrous elements projecting from said backing between said support regions;

When the backing material is heat treated to cause it to contract, the filaments form upstanding curved bridges between their points of interweaving engagement with the backing, thus providing the loop elements of the fastening device. The filaments are disposed in overlapping chevron configuration so that the raised loops cross each other in a zig-zag fashion to thereby provide better entanglement with the hook elements of the fastening device.

A similar type of loop element is disclosed by FR-A-1375254 in which a woven backing fabric formed of a mixture of heat shrinkable and non-heat-shrinkable fibers is subjected to heat treatment to provide raised loops suitable for engagement with the hook element of a hook and loop fastening device.

Such fastening devices have been found especially useful and favorable on disposable articles such as disposable garments, disposable diapers, disposable packages, cartons and the like. While such fastening devices provide a secure closing means, their use has, however, been limited on disposable articles due to the fact that such fastening devices are relatively costly. The major reason that such fastening devices are too costly is that they have high manufacturing costs. Thus, there is a need for a low cost fastening device for such disposable articles.

While many attempt have been made to provide a low-cost fastening device, most of the efforts have been directed toward developing a low-cost hook fastening material. The loop fastening material remains a costly element for a fastening device for disposable articles. Loop fastening materials typically have a number of woven loops extending outwardly from a backing. The loops may be provided by weaving a base fabric containing supplementary warp threads or by knitting. However, these processes produce generally costly loop fastening materials due to the fact that these processes are relatively slow.

Thus, it is an object of the present invention to provide a low cost and improved method for producing a loop fastening material forming part of a fastening device for disposable articles.

It is a further object of the present invention to provide a loop fastening material which may be formed by positioning a plurality of filaments on a backing to form a low-cost loop fastening material.

It is a further object of the present invention to provide a disposable diaper incorporating a hook and loop fastening device including the loop fastening material.

## SUMMARY OF THE INVENTION

According to the present invention there is provided a method for producing a loop component of a hook and loop fastening device for a disposable garment, said loop component having a multiplicity of fibrous elements projecting from a backing of orientable material capable of exhibiting a dimensionally unstable state and a dimensionally stable state, said backing having a predominant response direction in which the orientable material contract from its unstable state to its stable state, said method comprising the steps of:

(a) providing a backing of orientable material, in its unstable state;

(b) positioning a plurality of filaments on said backing so as to support and hold said filaments on said backing at spaced support regions disposed along each of said filaments, adjacent support regions of each filament defining therebetween an unsupported portion; and

(c) causing said orientable material to contract in its reponse direction to it stable state, thereby shirring said filaments at said unsupported portions to form fibrous elements projecting from said backing between said support regions;

wherein the backing of orientable material provided in step (a) comprises a continuous film of orientable material, and in that step (b) comprises bonding said filaments to said backing by means of adhesive, sealing or ultrasonic welding.

The present invention also relates to a disposable article incorporating a fastening device having a hook fastening material and a loop fastening material which comprises the improved loop fastening material of the present invention. The hook fastening material comprises any of the well known hook fastening materials as are known in the art and which have a base and a number of engaging elements extending from the base. The loop fastening material and the complementary hook fastening material provide a secure closing means that will resist shear stress and peel forcess encountered during use.

The present invention also relates more particularly to a disposable diaper having such an improved fastening device.

## BRIEF DESCRIPTION OF THE DRAWINGS

Figure 1 is a perspective view of the filaments and the backing used to form the present invention prior to the filaments being positioned or laid down on the backing.

Figure 2 is a perspective view of a loop fastening material of the present invention when the backing is in its dimensionally unstable state.

Figure 3 is a perspective view of a loop fastening material of the present invention when the backing is in its dimensionally stable state.

Figure 4 is a perspective view of an alternative embodiment of a loop fastening material of the present invention.

Figure 5 is a perspective view of a further alternative embodiment of a loop fastening material of the present invention.

Figure 6 is a perspective view of a still further alternative embodiment of a loop fastening material of the present invention.

Figure 7 is an enlarged fragmentary perspective view of the loop fastening material of Figure 6 showing the ultrasonic welds.

Figure 8 is a perspective view of a fastening device according to the present invention.

Figure 9 is an enlarged side view of an engaging element of a hook fastening material engaged with the fibrous elements of a loop fastening material of the present invention.

Figure 10 is an enlarged perspective view of a tape fastening system of a disposable diaper incorporating the loop fastening material of the present invention.

## DETAILED DESCRIPTION OF THE INVENTION

In the present invention, an orientable material is used as a backing for the loop fastening material. As used herein, the term "orientable material" refers to a web or film that has a dimensionally unstable state relative to some other dimensionally stable state and which can be caused to be transformed from the unstable to the stable state in a direction of response by the removal of forces acting on the web or film or by the application of any form of energy or by any other convenient treatment.

Orientable materials of the type useful in the present invention include a number of materials well known to those skilled in the art. For example, the polyurethane described in U.S. Patent 3,912,565 issued to Koch et al. on October 14, 1975; the heat recoverable material described in U.S. Patent 3,639,917 issued to Althouse on February 8, 1972; and the plasticized vinyl chloride described in U.S. Patent 3,819,401 issued to Massengale et al. on June 25, 1974 can be used as the backing in the present invention.

Since the most convenient and practical form of energy to transform the orientable material is heat, preferred orientable materials are heat-shrinkable materials having a dimensionally heat stable state and a dimensionally heat unstable state and which contract at least uni-directionally from the heat unstable to the heat stable state in a direction of response. This type of film is typically converted to a dimensionally heat unstable state via molecular orientation by laterally stretching it while at an elevated temperature which is less than its crystalline melting temperature and allowing it to cool. (This is generally done by means of a tentering apparatus.) When cooled, the polymers of the film are predominantly uni-axially oriented and the film essentially retains its new dimension until such time as it is again subjected to an elevated temperature where it recovers to its heat stable state. The film is thus in a heat unstable state. Specific film-forming compositions which are described as being suitable for forming webs capable of exhibiting heat stable and heat unstable states are disclosed in U.S. Patent No. 4,303,571 issued to Jansen et al. on December 1, 1981 and U.S. Patent No. 4,543,154 issued to G. Reiter on September 24, 1985.

Preferred heat-shrinkable materials include ethyl vinyl acetate copolymers, polystyrene-polyolefin block copolymers, ethylacrylate methacrylate copolymers and numerous other thermoplastic heat-sealable polymers as are known in the art. One particularly preferred heat-shrinkable material is a film comprising a blend of ethylene propylene rubber with ethyl vinyl acetate, such as that available from Exxon Chemical Company of Florham Park, New Jersey.

Alternatively, the orientable material of the backing may comprise an elastic or elastomeric material having an elongated orientation and a relaxed orientation and which contracts from its elongated orientation to its relaxed orientation in at least one predominant response direction. This type of material is typically converted to the elongated orientation by tensional forces applied in any manner along at least one axis of the film or web of the material, which, when the tensional forces are removed, contracts in its predominant response direction to its relaxed orientation, the material recovering a substantial proportion of its elongation. The elastomeric materials useful to form the backing thus include all suitable elastic materials capable of forming such an elastomeric backing. Elastomeric materials suitable for use herein include butadiene/acrylonitrile copolymers, styrene/isoprene copolymers, polyurethane elastomers, elastomeric films such as Kraton, natural rubber, or ethylene propylene-dimonomers.

The orientable materials of the present invention have been described in terms of having a dimensionally unstable state that is later transformed to its dimensionally stable state. While, in general, the dimensionally stable state is an absolute state, it is not necessary that it be. It is only required that the state following treatment be relatively more stable than the state preceeding treatment and that the state following treatment be sufficiently stable for practical use of the resultant loop fastening material.

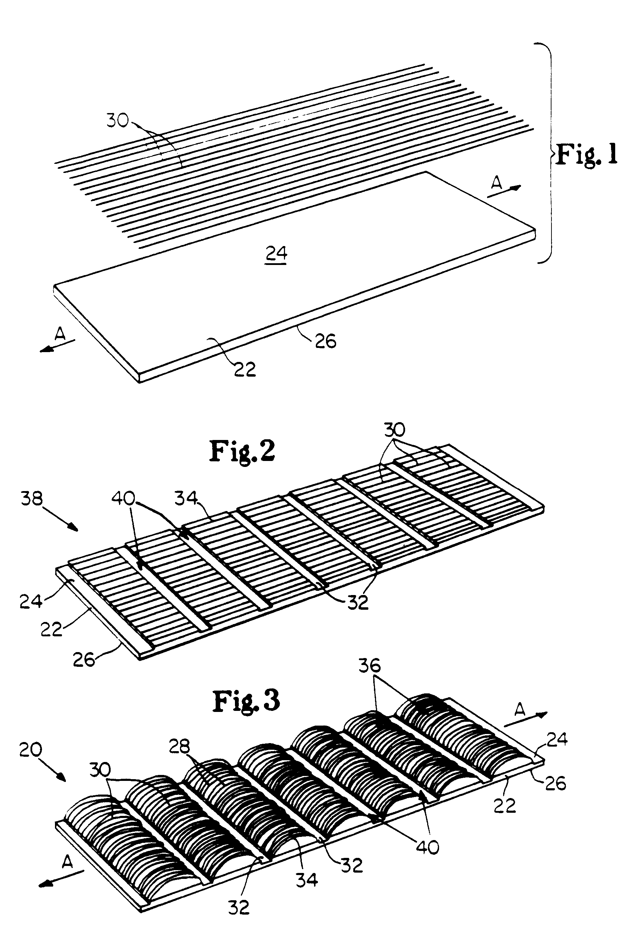

Referring to the drawings, Figure 3 shows a preferred embodiment, loop fastening material 20, of the present invention. As used herein, the term "loop fastening material" refers to the portion of a hook and loop-type fastening device that is designed to engage the engaging elements of a complementary hook fastening material. Thus, a loop fastening material may also be referred to as the female fastener, the loop fastener or the fibrous fastening material.

As shown in Figure 3, the loop fastening material 20 comprises a backing 22 of an orientable material, preferably a heat-shrinkable material shown in its heat stable state in Figure 3, having a first surface 24 and a second surface 26, and a multiplicity of fibrous elements 28 extending outwardly from the first surface 24 of the backing 22. The fibrous elements 28 are formed from filaments 30 positioned on and secured to the backing 22. The filaments 30 are intermittently secured to the backing 22 at spaced, support regions 32 along the length of each filament 30, while the heat shrinkable material of the backing 22 is in its heat unstable state, the support regions 32 defining between each pair an unsupported portion 34.

The filaments 30 ace preferably positioned on the backing 22 essentially parallel to the path of response of the orientable material, designated by the line A-A in Figure 3, and in parallel with each other. The filaments 30 are secured to the backing 22 across the filaments 30 and preferably along essentially the entire width of the backing 22 in a direction essentially perpendicular to the response direction and at regularly spaced intervals to form loop tunnels 36. Thus, each loop tunnel 36 preferably comprises a multiplicity of parallel fibrous elements 28.

Figure 1 shows the backing 22 and the filaments 30 used to form the loop fastening material 20 prior to their association. The heat shrinkable material used for the backing 22 is in its heat unstable state prior to the filaments 30 being positioned or laid down on the backing 22. (While the orientable material could be in its dimensionally stable state when the filaments 30 are positioned on the backing 22, this is not preferred because in causing the orientable material to be transformed to its unstable state, filaments 30 may be dislocated, disarranged, skewed or bonded into the backing 22 such that the fibrous elements would not be as effective in engaging the hook fastening material.) Thus, the filaments 30 ace preferably positioned on the backing 22 while the orientable material of the backing 22 is in its heat unstable state and while the filaments 30 are in an untensioned condition so that when the orientable material is caused to contract to its stable state, the filaments 30 are shirred at the unsupported portions 34 to form the fibrous elements 28 of the present invention. The filaments 30 are positioned on the backing 22 in a direction essentially parallel to the response direction of the heat-shrinkable material and preferably in parallel with each other.

Figure 2 shows a preferred embodiment of the loop fastening material 20 when the heat-shrinkable material is in its heat unstable state (dimensionally unstable state) and after the filaments 30 have been positioned on and secured to the backing 22. The filaments 30 are preferably intermittently secured to the backing 22 at the spaced, support regions 32 to form the insupported portions 34 therebetween. The filaments 30 are secured to the backing 22 in support regions 40 extending as bands across the filaments, and preferably across essentially the entire width of the backing 22, in a direction essentially perpendicular to the response direction of the heat-shrinkable material at regularly spaced intervals to form uniform width unsupported portions 34. As shown in Figures 2 and 3, the filaments 30 are secured to the backing 22 by ultrasonically welding the material together at unitary zones of attachment 40.

The resultant composite material 38 shown in Figure 2 is then caused to be transformed to its heat stable state (dimensionally stable state). Since the backing 22 is a heat-shrinkable material, the composite 38 is preferably heated, although the orientable material can be caused to be transformed to its dimensionally unstable state in many ways including irradiating the material or releasing the tension forces applied to the material. The heating of the composite 38 allows the heat-shrinkable material to contract to and regain its heat stable state in the response direction. As a result of the contraction or shrinkage of the backing 22, the filaments 30 are shirred or gathered at the unsupported portions 34 to form the fibrous elements 28 and the loop tunnels 36 as shown in Figure 3 extending from the first surface 24 of the backing 22.

The backing 22 of the loop fastening material 20 provides a strong base or foundation on or into which the filaments 30 are positioned and secured, and that can cause the filaments 30 to be shirred to form fibrous elements 28. Thus, in the present invention, the backing 22 comprises an orientable material capable of exhibiting a dimensionally stable state and a dimensionally unstable state and having a predominant response direction in which the orientable material is transformed from its unstable state to its stable state. While the backing 22 is shown in Figures 1 - 3 to preferably comprise only the orientable material, it should be understood that the backing 22 may also comprise any other suitable materials secured to the orientable material so long as such layer or layers of material are flexible so that they do not inhibit the shirring of the filaments 30 as the orientable material is allowed to contract to its stable state. Thus, for example, webs or films of material may be bonded, laminated or otherwise secured to the orientable material to form a composite backing so as to provide a stronger backing or a backing that is more compatible for bonding to the filaments or to the article onto which the loop fastening material 20 will be placed. Examples of such suitable materials include nonwovens, wovens, polyethylene films, or any other materials known in the art and suitable for such backing. As previously discussed, the backing 22 is preferably an ethyl vinyl acetate copolymer.

The fibrous elements 28 of the present invention securely engage the engaging elements of a complementary hook fastening material to provide a fastening device. The fibrous elements 28 each preferably comprise the loop structure shown in Figure 3 having a pair of spaced, support regions 32 and an unsupported portion 34 between the pair of support regions 32. While the fibrous elements 28 may have shapes other than the loop structure shown in Figure 3, such shapes are not preferred.

The fibrous elements 28 of the present invention are preferably formed by filaments 30 positioned on and secured to the backing 22. As used herein, the term "filament" defines a member having a high ratio of length to diameter or width. Thus, a filament may be a fiber, a thread, a strand, a yarn or any other member or combination of these members, including filaments that are preattached together in woven or nonwoven webs, as are known in the art. Suitable materials for such filaments include natural fibers such as cotton or wool; synthetic fibers of nylon, polyamides, polyesters or polyolefins; spun yarns; polyethylene fibers; polypropylene fibers: nylon fibers; non-woven webs: or any other material or combination of materials known in the art and suitable for use herein. It should be noted that in the preferred embodiment, the material used for the filaments 30 should not have a melting temperature lower than the temperature at which the orientable material is heat shrunk nor should the filaments 30 be heat-shrinkable at or below such temperature. In addition, the filaments 30 may be manufactured using a number of manufacturing techniques including those such that the filaments are spun, blown or the like. Preferably, each filament 30 is a polypropylene fiber of linear density 0.88 or 0.99 Tex (8 or 9 denier) included in a spunbounded nonwoven web such as manufactured by James River Corporation under the tradename Celestra.

The filaments 30 of the present invention are preferably continuous filaments. As used herein, the term "continuous filaments" refers to relatively long filaments that provide a minimum of loose ends in the central region of the backing. If the filaments 30 are not continuous filaments but are short or staple, then the ends of the staple filaments could be positioned as a portion of the unsupported portions 34 of the fibrous element 28 such that a complete fibrous element 28 having two support regions 32 and an unsupported portion 34 would not be formed. The resulting incomplete fibrous element 28 would not be able to securely engage the engaging elements of a hook fastening material such that the ability of the loop fastening material to provide a secure closure would be diminished. Thus, although short or staples fibers are contemplated for use in the present invention, continuous filaments 30 are preferred. A preferred continuous filament 30 has a length to make at least one complete fibrous element 28 and most preferably has a length such that the ends of the continuous filament 30 are either disposed at a support region 32 or adjacent the terminating edge of the backing 22.

While each fibrous element 28 is preferably formed from a single filament 30, the fibrous elements 28 may be formed from a segment of that filament 30 or the whole filament 30 depending upon the size, strength and manufacturing ease required to make the fibrous elements 28 to securely engage the hook fastening material. Thus, for example, the filament 30 may only have a pair of support regions 32 positioned adjacent its ends so that the fibrous element 28 is formed of a whole filament 30. Preferably, the filament 30 has a number of support regions 32 positioned along its length to form a plurality of fibrous elements 28 along each filament 30.

The filaments 30 are positioned on the backing 22 preferably while the orientable material of backing 22 is in the dimensionally unstable state and while the filaments 30 are in an untensioned condition. (While the filaments 30 could conceivably be positioned on the backing in a tensioned or unstable state, such is not preferred to provide for maximum shirring of the filaments.) The configuration in which the filaments 30 are positioned or laid down on the backing 22 determines the size and the ability of the loop fastening material 20 to provide an effective fastening device. While the filaments 30 may be randomly positioned on the backing 22 such that filaments 30 overlap or extend in many different directions, it has been found that the filaments 30 should preferably be positioned as parallel with each other as possible to provide fibrous elements 28 configured in a uniform direction. In addition, while the filaments 30 may be positioned lengthwise on the backing 22 in any direction, in order to take advantage of the maximum shirring effect of the orientable material of the backing 22 and to form fibrous elements 28 of maximum height, the filaments 30 are preferably positioned on the backing 22 in a direction essentially parallel to the predominant response direction of the orientable material. "Essentially parallel" is used herein to indicate that the filaments 30 need not extend absolutely parallel to the response direction so long as a majority of the filaments 30 extend parallel or a small deviation off of parallel to the response direction. The filaments 30 may be positioned or laid down on the backing 22 by any method or means as is known in the art.

Either after the filaments 30 have been positioned on the backing 22 or simultaneously with the filaments 30 being positioned on the backing 22, the filaments 30 are secured to the backing 22. The filaments 30 are secured to the backing 22, while the orientable material of the backing 22 is in its dimensionally unstable state, intermittently at spaced, support regions 32 along the lenght of the filaments 30 and preferably as continuous bands extending across a number or all of the filaments 30 (across the width of the backing 22). The filaments 30 may be secured to the backing 22 by any method or means as is known in the art. For example, the filaments 30 may be adhesively secured with hot melt adhesives, glues, pressure sensitive adhesives or any other adhesive material as is known in the art. In addition, the filaments 30 may be heat/pressure sealed into the backing 22. Preferably, the filaments 30 ace ultrasonically welded to the backing 22 by ultrasonic means as are known in the art. A method of and apparatus for ultrasonically welding two webs together is described in U.S. Patent 4,531,999 issued to Persson et al. on July 30, 1985.

The filaments 30 are secured to the backing 22 intermittently along the length of each of the filaments to form the spaced, support regions 32. Intermittent securement being used to defined alternating regions of securement (support regions 32) and nonsecurement (unsupported portions 34) at either regularly or irregularly spaced intervals.

The support regions 32 can be spaced apart at intervals of any dimension depending upon the size of the engaging elements of the hook fastening material and the height of the fibrous element 28 required to provide the necessary shear and peel resistance. They are preferably regularly spaced, but they can be irregularly spaced. They are preferably regularly spaced at intervals of from 0.5 mm to 25 mm, more preferably from 2 mm to 5 mm, as measured from center to center when the orientable material is in its heat stable state.

While each individual filament 30 could be secured to the backing 22 at support regions 32 along the filament 30 that are positioned at intervals different from the support regions of each adjacent filament, as shown in Figures 2 and 3 the filaments 30 are preferably secured so that the support regions extend as a band across a plurality of or all of the filaments 30. Thus a number of adjacent filaments 30 are secured to the backing 22 along a line (straight or curvilinear) across the filaments (across the width of the filaments) so that the support regions of one filament are adjacent the support regions of an adjacent filament. The term "across the filaments" refers to an orientation generally in the direction of the width of the filaments or transverse to the length of the filaments. A band of securement is preferred so that the fibrous elements 28 formed are uniform and so that the loop tunnels 36 of the present invention are formed. Such securement is also preferred because such a loop fastening material 20 is less expensive to produce because of the speed increase obtainable in manufacturing such a loop fastening material. The filaments 30 may be secured to the backing in any pattern or combination of patterns to provide loop tunnels 36 of differing lengths, widths or a pattern of varying loop tunnels 36. The filaments 30 are preferably secured across the filaments in at least spaced segments and most preferably across essentially the entire width of the backing 22 for ease of manufacture and uniformity of the loop tunnels 36. The term "across essentially the entire width" is used in this context to indicate that the continuous bonds need not extend absolutely across the entire width of the backing 22.

The filaments 30 are also preferably secured in a straight line across the filaments 30 in a direction essentially perpendicular to the predominant response direction of the backing 22. The term "essentially perpendicular" to the response direction refers to an orientation generally perpendicular but not absolutely perpendicular to the response direction. As illustrated, the support regions 32 are shown to be positioned at essentially right angles to the longitudinal dimension of the backing 22. One can, however, depart from the perpendicular orientation without departing from the scope and spirit of this invention. Thus, the filaments 30 may be secured at an angle to the perpendicular. However, it is believed that the departure from the perpendicular may become too great for practical operation of the present invention when the angle from the perpendicular exceeds about 45°. In Figures 2 and 3, the filaments 30 are preferably shown as being secured at regularly spaced, support regions 32 positioned essentially perpendicular to the predominant response direction of the backing 22.

The filaments 30 are preferably ultrasonically welded to the backing 22. The ultrasonic welding pattern may comprise unitary zones of attachment or discrete spaced zones of attachment. For example, the spaced zones of attachment could be circular or elliptical shapes as are shown in U.S. Patent 4,515,595 issued to Kievit et al. on May 7, 1985. Preferably, however, the welding pattern comprises unitary zones of attachment 40 as are shown in Figures 2 and 3.

An exemplary embodiment of the loop fastening material 20 comprises a backing 22 of a material of a blend of ethylene propylene rubber with ethyl vinyl acetate having a multiplicity of continuous 0.99 Tex (9 denier), polypropylene filaments 30 positioned on and secured to the backing 22. The filaments 30 are positioned parallel to each other and in a direction essentially parallel to the response direction of the backing 22. The filaments 30 are ultrasonically welded to the backing 22, while the backing 22 is in its heat unstable state, at unitary zones of attachment 40 at regularly spaced intervals of 2.5 mm (about 0.1 inches) along the filaments 30 and across the filaments along the entire width of the backing 22 in a straight line positioned at an angle that is essentially perpendicular to the response direction. After the resultant composite material has been heated to cause the heat shrinkable material to contract along its path of response to its heat stable state, the filaments 30 are shirred at the unsupported portions 34 to form a multiplicity of fibrous elements 28 and a plurality of loop tunnels 36 on the backing 22.

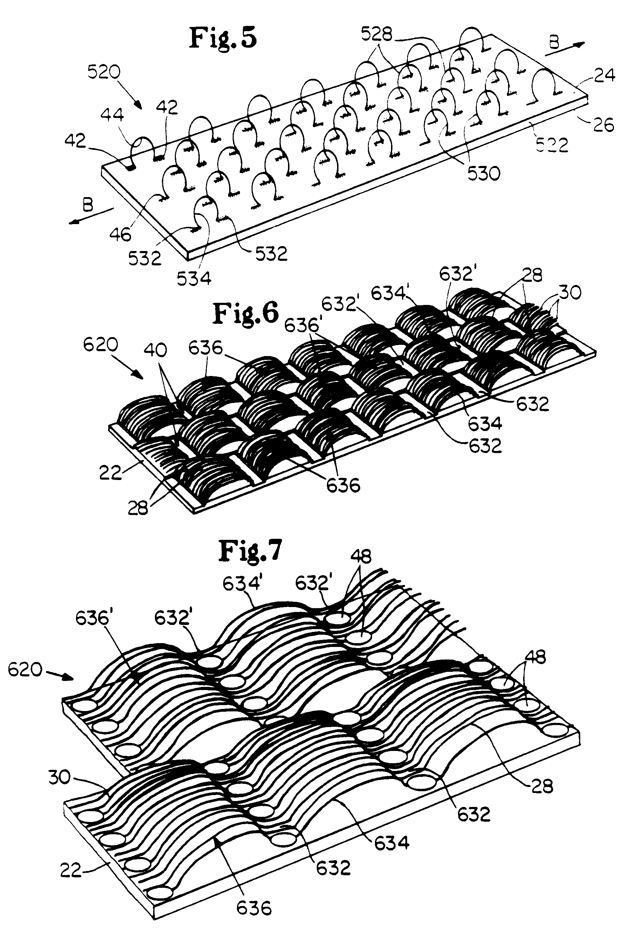

Figure 4 shows an alternative embodiment, loop fastening material 420, of the present invention. The loop fastening material 420 of Figure 4 is similar to that shown in Figure 3 except that the filaments 30 are continually secured at an angle to the perpendicular of the response direction of the orientable material. Such a loop fastening material 420 thus has a number of loop tunnels 436 disposed on the backing 22 at an angle to the edges of the backing 22. Such a loop fastening material 420 is believed to be especially effective for engaging hook fastening materials wherein the engaging elements are disposed in a regularly spaced pattern such that a complete row of engaging elements engage different loop tunnels 436 and are not disposed completely at a support region 32.

Figure 5 shows an alternative embodiment, loop fastening material 520, of the present invention. The backing 522 of the loop fastening material 520 comprises an elastomeric material such as an elastic member of natural rubber such as is marketed by Easthampton Rubber Thread Company. The elastomeric material has an elongated orientation (not shown), a relaxed orientation (shown in Figure 5), and a predominant response direction, line B-B, along which the elastomeric material contracts when tension is released from the backing 522. The fibrous elements 528 of the loop fastening material 520 each comprise a whole filament 530 in which the spaced, support regions 532 are located adjacent the ends 42 of the filament 530 such that the unsupported portion 534 comprises the middle portion 44 of the filament 530. A preferred filament 530 used for the loop fastening material 520 is a spun thread of a cotton blend such as an ordinary sewing thread as is well known.

The loop fastening material 520 is formed by applying tensional forces to the backing 22 in any manner along the axis of the desired direction of response and then positioning and securing the filaments 530 to the backing 522 while the backing 522 is in this elongated orientation. Each of the filaments 530 are randomly positioned on the backing 522 in a preferred direction essentially parallel to the direction of response. The filaments 530 are adhesively secured on the backing 522 at the support regions 532 by applying a hot melt adhesive 46 to the ends of the filaments 530. After the adhesive 46 is allowed to set up, the tensional forces are removed and the backing 522 is allowed to contract to its relaxed orientation, the fibrous elements 528 being formed by the shirring of the filaments 530 at the unsupported portions 534. After the fibrous elements 528 are formed, the elastomeric material is preferably stabilized so that the elastomeric material will resist re-elongation during use that would otherwise distort the fibrous elements 528. The elastomeric material may be stabilized by any means or by a method such as by attaching it to a relatively nonelastic member or material.

Figure 6 shows a further alternative embodiment, loop fastening material 620, of the present invention. The loop fastening material 620 has a plurality of loop tunnels 636 and 636′ disposed in a discrete spaced pattern across the width of the backing 22. In the particular embodiment, the loop tunnels are positioned in an evenly staggered pattern wherein the support regions 632 of one row of loop tunnels 636 are positioned in the center of the unsupported portions 634′ of the adjacent rows of loop tunnels 636′.

Figure 7 shows an enlarged view of a portion of the loop fastening material 620 while the backing 22 is in its heat stable state. As shown, the filaments 30 are ultrasonically welded to the backing 22 at spaced evenly staggered, support regions 632 and 632′. The support regions 632 and 632′ are evenly spaced such that the support regions 632 and 632′ are evenly staggered. The support regions 632 and 632′ are ultrasonically welded at discrete zones of attachment 48 in a circular shape.

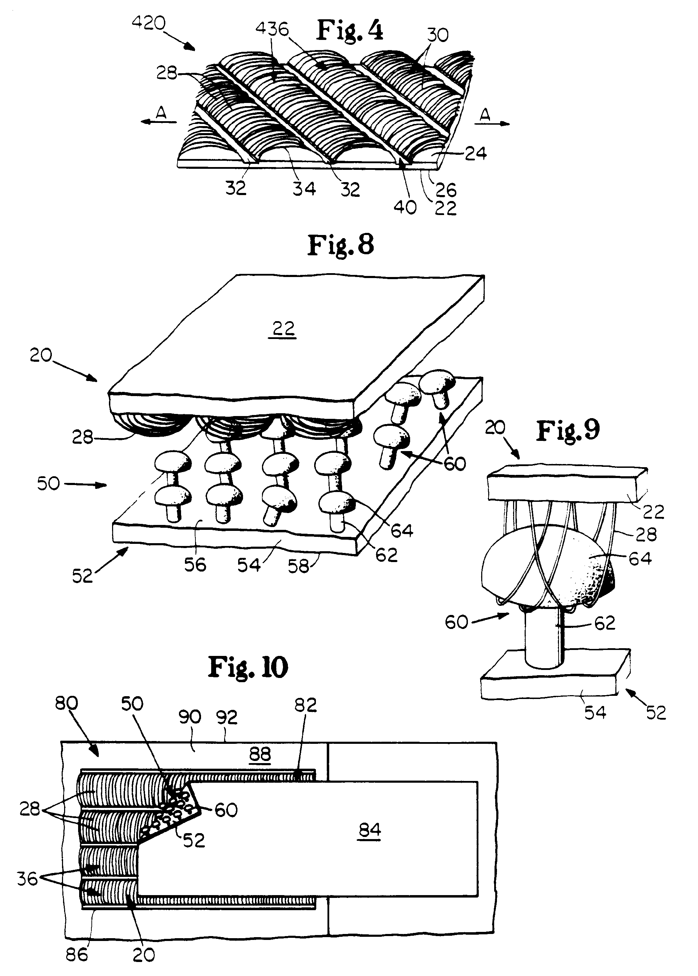

Figure 8 shows a preferred fastening device 50 of the present invention. The fastening device 50 preferably comprises the loop fastening material 20 of the present invention and a complementary hook fastening material 52 engageable with the fibrous elements 28 of the loop fastening material 20. As used herein, the term "hook fastening material" is used to designate the portion of the fastening device 50 having engaging elements. Thus, the hook fastening material may also be referred to as the male fastener. It should also be understood that the use of the term "hook" should be nonlimiting in the sense that the engaging elements may comprise any shape as is known in the art so long as they are adapted to engage a complementary loop fastening material. As shown, the hook fastening material 52 preferably comprises a base 54 having a first surface 56 and a second surface 58 and a plurality of engaging elements 60 extending from the first surface 58 of the base 54. Each of the engaging elements 60 are shown to preferably comprise a stem 62 supported at one end on the base 54 and an enlarged head 64 positioned at the end of the stem 62 opposite of the base 54.

The hook fastening material 52 may be manufactured from a wide range of materials. Such suitable materials include nylon, polyester, polypropylene or any combination of these or other materials. A suitable hook fastening material 52 comprises a number of shaped engaging elements projecting from a woven backing such as the commercially available material designated "Scotchmate" brand No, FJ3402 available from Minnesota Mining and Manufacturing Company, St. Paul, Minnesota. Alternatively, the engaging elements may have any other shape such as hooks, "T's", or any other shape as are well known in the art. A particularly preferred hook fastening material is described in copending European Patent Application No. 88300605.8 entitled "Disposable Diaper Having An Improved Fastening Device" filed January 26, 1988, Publication N° 0276970.

In use, the hook and loop fastening materials are pressed face to face against each other so that the fibrous elements 28 of the loop fastening material 20 "catch" on the engaging elements 60 of the hook fastening material 52. With the fibrous elements 28 thus "hooked" or caught by the engaging elements 60 as is shown in Figure 9, the connection between the elements resists sheer stress and certain peel forces applied to the fastening device 50. The fastening device 50 is separated by peeling the hook fastening material 52 away from the loop fastening material 20 such that the fibrous elements 28 are released, or broken, or the bonds of the support regions 32 break. Thus, the hook fastening material 52 is completely detached from the loop fastening material 20.

The fastening device 50 of the present invention has been found to be especially useful for disposable articles. The loop fastening material can be made relatively inexpensively in comparison to the woven loops used in known fastening devices. In addition, since the fastening device on a disposable article is opened and closed far fewer times than on reuseable articles, hte loop fastening material is more suited for disposable articles since it need only strong enough to provide a limited number of secure closures (10-20 times). It should be noted, however, that the loop fastening material can be made much stronger for use on durable articles or for any other contemplated use by, for example, increasing the diameter or denier of the filaments, more strongly securing the filaments to the backing or increasing the density of the fibrous elements relative to the number of engaging elements. (However, these changes also increase the cost of such loop fastening materials.) Thus, the fastening device 50 is especially useful on such disposable articles as packaging, disposable absorbent articles, disposable wraps or any other disposable material.

As shown in Figure 10, the fastening device 50 is preferably positioned on a disposable absorbent article such as a disposable diaper 80. The disposable diaper 80 preferably comprises a liquid pervious topsheet, an absorbent core, a liquid impervious backsheet and elastic members. While the topsheet, the absorbent core, the backsheet and the elastic members may be assembled in a variety of well known configurations, a preferred disposable diaper configuration is described in U.S. Patent 3,860,003 entitled "Contractible Side Portions For Disposable Diapers" which issued to K. B. Buell on January 14, 1975.

As shown in Figure 10, the tape fastening system 82 of the diaper 80 preferably comprises the fastening device 50 of the present invention. Any of the well known configurations and constructions may be used as the tape fastening system. A preferred tape fastening system is a Y-tape as described in detail in U.S. Patent 3, 848, 594 entitled "Tape Fastening System For Disposable Diaper" which issued to K. B. Buell on November 19, 1974. Alternatively preferred tape fastening systems are described in detail in co-pending published European Patent Application Nos 0233704 and 0240213; and the previously mentioned co-pending European Patent Application No. 88300605. 8.

The preferred tape fastening system illustrated in Figure 10 has a tape tab 84 and a second member 86.

Preferably, the tape tab 84 comprises the hook fastening material 52 having the engaging elements 60 extending from the tape tab 84. The second member 86 preferably is disposed on the outside surface 88 of the waist portion 90 of the diaper 80, in the preferred embodiment of the disposable diaper 80, the loop fastening material 20 of the present invention comprises the second member 86. As shown, the loop fastening material 20 preferably is oriented on the second waist portion 90 of the diaper 80 with the loop tunnels 36 extending essentially parallel to the end edge 92 of the diaper 80. (Parallel to the direction of shear forces applied to the fastening device during use.) Thus, in this configuration the fibrous elements 28 provide the maximum peel and shear force resistance. The loop fastening material 20 may, however, be oriented on the second waist portion 90 in any configuration such as with the loop tunnels 36 extending perpendicular to the end edge 92 of the diaper 80 or at any angle to the end edge 92.

## Claims

1. A method for producing a loop component (20, 420, 520, 620) of a hook and loop fastening device for a disposable garment, said loop component having a multiplicity of fibrous elements (28, 528) projecting from a backing (22, 522) of orientable material capable of exhibiting a dimensionally unstable state and a dimensionally stable state, said backing (22, 522) having a predominant response direction in which the orientable material contracts from its unstable state to its stable state, said method comprising the steps of:
   (a) providing a backing (22, 522) of orientable material, in its unstable state;
   (b) positioning a plurality of filaments (30,530) on said backing so as to support and hold said filaments on said backing at spaced support regions (32, 532, 632) disposed along each of said filaments (30,530), adjacent support regions (32, 532, 632) of each filament defining therebetween an unsupported portion (34, 534, 634); and
   (c) causing said orientable material to contract in its reponse direction to its stable state, thereby shirring said filaments (30, 530) at said unsupported portions (34, 534, 634) to form fibrous elements (28, 528) projecting from said backing between said support regions;
characterised in that
the backing of orientable material provided in step (a) comprises a continuous film of orientable material, and in that step (b) comprises bonding said filaments to said backing by means of adhesive, sealing or ultrasonic welding.

2. A method according to Claim 1 wherein said filaments (30, 530) are disposed substantially parallel to said predominant response direction of said film.

3. A method according to either one of Claims 1 & 2 wherein said filaments (30, 530) are secured to said backing (22, 522) in support regions (32, 632, 632') extending as a band (40) across said filaments, either in spaced segments (632, 632') or across essentially the entire width of said backing, in a direction essentially perpendicular to the predominant response direction of said orientable material or at an angle to the perpendicular to the predominant reponse direction of said orientable material.

4. A method according to any one of Claims 1-3 wherein each of said filaments (30) forms a plurality of fibrous elements (28).

5. A method according to any one of Claims 1-4 wherein said continuous film of orientable material provided in step (a) comprises a film of heat shrinkable material, preferably a film of ethylene vinyl acetate copolymer.

6. A method according to any one of Claims 1-4 wherein the continuous film of orientable material provided in step (a) comprises an elastomeric film to which tensional forces have been applied to stretch it to its elongated orientation.

7. A method according to any one of Claims 1-6 wherein step (c) comprises irradiating the orientable material to cause it to contract to its dimensionally stable state, heating the heat shrinkable material to cause it to contract along its predominant response direction to its heat stable state, or releasing the tensional forces applied to the elastomeric material to cause the elastomeric material to contract along its response direction to its relaxed orientation.

8. A disposable absorbent article incorporating a hook and loop fastening device (50) wherein the loop component (20) is made in accordance with any one of claims 1-7.

9. A disposable absorbent article according to Claim 8 comprising a disposable diaper (80) formed with a liquid pervious top sheet, a liquid impervious backsheet, an absorbent core disposed therebetween, and elastic members adapted to contract side edge portions of said diaper.

10. A disposable diaper according to Claim 9 wherein the hook and loop fastening device (50) comprises a tape tab (84) and a second member (86) interengageable with said tab (84), said second member (86) comprising the loop component (20) and being disposed on the backsheet forming the outer surface (88) of a waist portion (90) of the diaper (80).

## Patentansprüche

1. Ein Verfahren zur Herstellung eines Schlaufen-Bestandteiles (20, 420, 520, 620) einer Haken- und Schlaufen-Befestigungseinrichtung für ein wegwerfbares Kleidungsstück, wobei der genannte Schlaufen-Bestandteil eine Vielzahl von Faserelementen (28, 528) aufweist, welche von einem Träger (22, 522) aus ausrichtbarem Material abragen, welches fähig ist, einen dimensional instabilen Zustand und einen dimensional stabilen Zustand aufzuweisen, wobei der genannte Träger (22, 522) eine dominante Reaktionsrichtung aufweist, in welcher das ausrichtbare Material von seinem instabilen Zustand zu seinem stabilen Zustand kontrahiert, wobei das genannte Verfahren die Schritte aufweist:

(a) Beistellen eines Trägers (22, 522) aus ausrichtbarem Material, in dessen instabilem Zustand;

(b) Positionieren einer Vielzahl von Fäden (30, 530) am genannten Träger, um so die genannten Fäden auf dem genannten Träger an beabstandeten Stützbereichen (32, 532, 632), welche entlang jedem der genannten Fäden (30, 530) angeordnet sind, zu stützen und zu halten, wobei benachbarte Stützbereiche (32, 532, 632) eines jeden Fadens dazwischen einen ungestützten Abschnitt (34, 534, 634) definieren; und

(c) Veranlassen des genannten ausrichtbaren Materials, in seiner Reaktionsrichtung zu seinem stabilen Zustand zu kontrahieren, wobei dadurch die genannten Fäden (30, 530) an den genannten ungestützten Abschnitten (34, 534, 634) gekräuselt werden, um Faserelemente (28, 528) zu bilden, welche vom genannten Träger zwischen den genannten Stützbereichen abragen;

dadurch gekennzeichnet,

daß der in Schritt (a) beigestellte Träger aus ausrichtbarem Material eine kontinuierliche Folie aus ausrichtbarem Material umfaßt, und dadurch, daß Schritt (b) die Bindung der genannten Fäden an den genannten Träger mittels Klebstoff/ Einschmelzen oder Ultraschallschweißen enthält.

2. Ein Verfahren nach Anspruch 1, bei welchem die genannten Fädern (30, 530) im wesentlichen parallel zur genannten dominanten Reaktionsrichtung der genannten Folie angeordnet sind.

3. Ein Verfahren nach einem der Ansprüche 1 und 2, bei welchem die genannten Fäden (30, 530) am genannten Träger (22, 522) in Stützbereichen (32, 632, 632′), welche sich als ein Band (40) über die genannten Fäden erstrecken, befestigt sind, entweder in beabstandeten Segmenten (632, 632′) oder über im wesentlichen die Gesamtbreite des genannten Trägers, in einer Richtung im wesentlichen lotrecht zur dominanten Reaktionsrichtung des genannten ausrichtbaren Materials.

4. Ein Verfahren nach einem der Ansprüche 1 bis 3, bei welchem jeder der genannten Fäden (30) eine Vielzahl von Faserelementen (28) bildet.

5. Ein Verfahren nach einem der Ansprüche 1 bis 4, bei welchem die genannte kontinuierliche Folie aus in Schritt (a) beigestelltem ausrichtbaren Material eine Folie aus schrumpfbarem Material, vorzugsweise eine Folie von Äthylen-Vinyl-Acetat-Compolymer, umfaßt.

6. Ein Verfahren nach einem der Ansprüche 1 bis 4, bei welchem die kontinuierliche Folie aus in Schritt (a) beigestelltem ausrichtbaren Material eine Elastomerfolie umfaßt, auf welche Zugkräfte aufgebracht worden sind, um sie in ihre verlängerte Ausrichtung zu strecken.

7. Ein Verfahren nach einem der Ansprüche 1 bis 6, bei welchem Schritt (c) ein Bestrahlen des ausrichtbaren Materials, um es zum Kontrahieren zu seinem dimensional stabilen Zustand zu veranlassen, ein Erhitzen des schrumpfbaren Materials, um es zum Kontrahieren entlang seiner dominanten Reaktionsrichtung zu seinem wärmebeständigen Zustand zu veranlassen, oder ein Lösen der auf das Elastomermaterial aufgebrachten Zugkräfte umfaßt, um das Elastomermaterial zu veranlassen, entlang seiner Reaktionsrichtung zu seiner entspannten Ausrichtung zu kontrahieren.

8. Ein wegwerfbarer absorbierender Artikel, welcher eine Haken- und Schlaufen-Befestigungseinrichtung (50) enthält, bei welcher der Schlaufen-Bestandteil (20) in Übereinstimmung mit irgendeinem der Ansprüche 1 bis 7 hergestellt worden ist.

9. Ein wegwerfbarer absorbierender Artikel nach Anspruch 8, welcher eine Wegwerfwindel (80) umfaßt, welche mit einem flüssigkeitsdurchlässigen Deckblatt, einem flüssigkeitsundurchlässigen Rückenblatt, einem dazwischen angeordneten absorbierenden Kern und elastischen Bauteilen ausgebildet ist, welche adaptiert sind, um Seitenrandabschnitte der genannten Windel zu kontrahieren.

10. Eine Wegwerfwindel nach Anspruch 9, bei welcher die Haken- und Schlaufen-Befestigungseinrichtung (50) eine Bandlasche (84) und einen zweiten Bauteil (86) enthält, welcher mit der genannten Lasche (84) ineinandergreifen kann, wobei der genannte zweite bauteil (86) den Schlaufen-Bestandteil (20) enthält und am Rückenblatt, welches die äußere Oberfläche (88) eines Taillenabschnittes (90) der Windel (80) bildet, angeordnet ist.

**Revendications**

1. Un procédé de fabrication d'un composant à boucles (20, 420, 520, 620) d'un dispositif d'attache ou de fermeture à boucles et à crochets pour un article de vêtement à jeter après usage ledit composant à boucles comprenant une pluralité d'éléments en fibres (28, 528) faisant saillie à partir d'un support (22, 522) en matériau orientable, susceptible de présenter un état dimensionnellement instable et un état dimensionnellement stable, ledit support (22, 522) possédant une direction de réponse prédominante dans laquelle le matériau orientable se contracte à partir de son état instable jusqu'à son état stable, ledit procédé comprenant les étapes:

(a) de founiture d'un support (22, 522) en matériau orientable à l'état instable;

(b) de positionnement d'une pluralité de filaments (30, 530) sur ledit support de manière à supporter et à maintenir lesdits filaments sur ledit support dans des zones de support espacées (32, 532, 632) disposées le long de chacun desdits filaments (30, 530), des zones de support adjacentes (32, 532, 632) de chaque filament définissant entre elles une zone non supportée (34, 534, 634); et

(c) de contraction dudit matériau orientable dans sa direction de réponse, à l'état stable, de manière à froncer ainsi lesdits filaments (30, 530) dans lesdites zones non supportées (34, 534, 634) pour former des éléments en fibres (28, 528) faisant saillie à partir dudit support entre lesdites zones de support;

caractérisé en ce que

le support en matériau orientable fourni à l'étape (a) comprend un film continu de matériau orientable, et en ce que l'étape (b) comprend la fixation desdits filaments sur ledit support par adhésif, scellement ou soudage par ultrasons.

2. Un procédé selon la revendication 1, dans lequel lesdits filaments (30, 530) sont disposés sensiblement parallèlement à ladite direction de réponse prédominante dudit film.

3. Un procédé selon l'une quelconque des revendications 1 et 2, dans lequel lesdits filaments (30, 530) sont fixés audit support (22, 522) dans des zones de support (32, 632, 632') s'étendant en forme de bande (40) transversalement auxdits filaments, soit en segments espacés (632, 632'), soit transversalement, sensiblement sur la largeur entière dudit support, dans une direction sensiblement perpendiculaire à la direction de réponse prédominante dudit matériau orientable ou formant un angle par rapport à la perpendiculaire à la direction de réponse prédominante dudit matériau orientable.

4. Un procédé selon l'une quelconque des revendications 1 à 3, dans lequel chacun desdits filaments (30) forme une pluralité d'éléments fibreux (28).

5. Un procédé selon l'une quelconque des revendications 1 à 4, dans lequel ledit film continu de matériau orientable prévu à l'étape (a) comprend un film continu de matériau thermo-rétractable, de préférence un film continu de copolymère éthylène-acétate de vinyle.

6. Un procédé selon l'une quelconque des revendications 1 à 4, dans lequel le film continu en matériau orientable fourni à l'étape (a) comprend un film élastomère auquel des forces de tension ont été appliquées pour l'étirer à son orientation allongée.

7. Un procédé selon l'une quelconque des revendications 1 à 6 , dans lequel l'étape (c) comprend l'irradiation du matériau orientable pour provoquer sa contraction à son état dimensionnellement stable, le chauffage dudit matériau thermo-rétractable pour provoquer sa contraction le long de sa direction de réponse prédominante, à son état thermique stable, ou la relaxation des forces de tension appliquées au matériau élastomère pour provoquer la contraction du matériau élastomère le long de sa direction de réponse à son orientation de relaxation.

8. Un article absorbant à jeter après usage comprenant un dispositif d'attache ou de fermeture à boucles et à crochets (50) dans lequel le composant à boucles (20) est réalisée selon l'une quelconque des revendications 1 à 7.

9. Un article absorbant à jeter après usage selon la revendication 8 comprenant une couche pour bébé à usage unique (80) formée avec une feuille supérieure perméable aux liquides, une feuille inférieure imperméable aux liquides, une partie centrale absorbante disposée entre les deux, et des éléments élastiques agencés pour contracter les parties latérales de bordure de ladite couche.

10. Une couche à jeter après usage selon la revendication 9, dans laquelle le dispositif d'attache ou de fermeture à boucles et à crochets (50) comprend une patte formant ruban (84) et un second élément (86) venant mutuellement en prise avec ladite patte (84), ledit second élément (86) comprenant le composant à boucles (20) et étant disposé sur la feuille de fond formant la surface extérieure (88) d'une partie de ceinture (90) de la couche (80).

**Fig. 1**

30

A

24

A

26

22

**Fig. 2**

30

38

40    34

24

22

26

32

**Fig. 3**

36

A

28

24

20

30

26

22

40

32

34

32

32

# Fig.5

# Fig.6

# Fig.7

Fig. 4

Fig. 8

Fig. 9

Fig. 10